**Europäisches Patentamt**

(19) **European Patent Office**

**Office européen des brevets**

(11) Publication number : **0 263 125 B1**

(12) # EUROPEAN PATENT SPECIFICATION

(45) Date of publication of patent specification :
**13.05.92 Bulletin 92/20**

(51) Int. Cl.[5] : **C12M 1/40**, G01N 33/48

(21) Application number : **87901601.2**

(22) Date of filing : **19.03.87**

(86) International application number :
**PCT/GB87/00192**

(87) International publication number :
**WO 87/05624 24.09.87 Gazette 87/21**

(54) **BIOCHEMICAL DETECTOR.**

(30) Priority : **19.03.86 GB 8606824**

(43) Date of publication of application :
**13.04.88 Bulletin 88/15**

(45) Publication of the grant of the patent :
**13.05.92 Bulletin 92/20**

(84) Designated Contracting States :
**AT BE CH DE FR GB IT LI LU NL SE**

(56) References cited :
**EP-A- 096 095**
**EP-A- 0 121 385**
**FR-A- 1 479 929**
**FR-A- 2 047 047**
**FR-A- 2 228 839**
**FR-A- 2 282 114**
**US-A- 4 334 880**

(73) Proprietor : **University of Strathclyde**
**McCance Building, 16 Richmond Street**
**Glasgow G1 1YQ, Scotland (GB)**

(72) Inventor : **SUCKLING, Colin, James**
**62 North Grange Road**
**Bearsden Glasgow G61 3AF (GB)**
Inventor : **PETHRICK, Richard, Arthur**
**40 Langside Drive**
**Glasgow G43 2QQ (GB)**

(74) Representative : **Szczuka, Jan Tymoteusz et al**
**Cruikshank & Fairweather 19 Royal Exchange**
**Square**
**Glasgow G1 3AE Scotland (GB)**

EP 0 263 125 B1

Jouve, 18, rue Saint-Denis, 75001 PARIS

## Description

The present invention relates to a device for use in the detection of biochemical analytes in liquid samples.

The regular monitoring of various biochemical analyte levels in body fluids is extremely important in the management of certain conditions as for example in the case of diabetes where various methods have been previously proposed for measuring glucose levels in solutions thereof. In one such method an enzyme catalysed reaction of glucose is monitored via changes in fluorescence spectra resulting from the reaction. In another method an oxygen electrode is provided with a glucose oxidase enzyme coating and the reduction in the level of oxygen reaching the electrode due to consumption thereof by an enzyme catalysed reaction of glucose with oxygen in said coating is used to obtain an indication of the amount of oxygen reaching the electrode. In both cases it will be noted that the methods employed involve the use of relatively cumbersome and/or specialized and expensive apparatus thereby limiting the circumstances under which glucose determinations can be carried out.

European patent specification EP 0096095 and U.S. patent specification US 4334 880 disclose a semi-conductor device, which comprises a strip of semiconductive organic polymer (such as a polypyrrole), which has been soaked in a binding agent (such as an antibody or an enzyme) having an affinity for the analyte being detected.

It is an object of the present invention to avoid or minimize one or more of the above disadvantages.

The present invention provides a device for use in the detection, in a liquid sample, of an analyte which is reactive under predetermined enzyme-catalysed conditions so as to produce at least one of a proton and hydrogen peroxide, which device is in the form of a multilayer thin film device and comprises:

(1) an electrically conducting thin film polymer element which comprises a polymer including in its polymeric structure monomer-derived units having conjugatable electron systems and protonatable N atoms such that the polymer can exist in a protonated or deprotonated form; and being permeable to protons, and being permeable to hydrogen peroxide where said analyte under said enzyme-catalysed conditions produces hydrogen peroxide; and said electrically conducting polymer element having an electrical conductivity in the range of from $10^{-4}$ to $10^2$ S cm$^{-1}$ and which is variable according to the amount of protons or the amount of hydrogen peroxide in contact with said polymer element, in use the electrically conducting polymer element being initially in the reduced deprotonated form prior to contact with said liquid sample;

(2) electrical connection means being electrically-conductively attached to said electrically conducting polymer element, the electrical connection means being spaced apart one from another and being intended in use of the device for electrically connecting said electrically conducting polymer element to external electrical circuit means to allow for directly or indirectly detecting changes in electrical resistance of said electrically conducting polymer element;

(3) an enzyme support element containing at least one enzyme, and additionally containing any cofactor(s) for said at least one enzyme which are required in said predetermined enzyme catalysed conditions, in an analyte-permeable substantially electrically non-conducting highly cross-linked polymeric water-swellable gel; the enzyme support element having a first face which is in contact with said electrically conducting polymer element, and having a second face which in use of the device may be brought indirectly into contact with said liquid sample containing said analyte to be detected; and

(4) a filter means selectively permeable to the analyte which is disposed at said second face for screening said enzyme support element from at least one other component of said liquid sample and which is substantially impermeable to at least one of proteinaceous material and macromolecular substances, said filter means comprising a highly cross-linked polymeric water-swellable gel.

Desirably in the case of a device of the invention based on the production and detection of protons, the polymer is reduced prior to immersion in the unknown analyte containing solution in order to increase the sensitivity of the device. Reduction may be effected by any suitable means but conveniently is effected by immersion in an aqueous alkaline solution such as dilute sodium hydroxide.

With a device of the present invention the amount of analyte present is obtained as a change in electrical conductivity which can be simply measured by connecting to said connection means any of a variety of readily available and/or relatively inexpensive means such as an ohm meter or resistance bridge circuit. It may moreover be noted that unlike certain previous systems, the device of the present invention does not in general require the use of any special environmental operating conditions such as anaerobic conditions.

The device may be made in various shapes and sizes but will be in the form of a multilayer thin film device made up of a thin film polymer element coated on one or both sides with the enzyme support element. Where only one side is coated with the support element then the other side will normally be sealed with an impermeable coating to avoid extraneous interactions with the polymer element. The filter means will generally be in the form of a further coating on top of the enzyme support element coating.

The connection means are normally in the form of highly conductive metal electrodes e.g. of platinum, silver, gold or copper, extending into the polymer element. Conveniently these electrodes can be used as a support for the polymer element, the latter being in the form of a film coating encasing part of the electrodes.

Various electrically conducting polymers containing conjugated monomer units may be used for the polymer element. Suitable polymers will have a conductivity in the range from $10^{-4}$ to $10^2$S cm$^{-1}$. Particular polymers that may be mentioned include polypyrrole and polymers based on aniline and pyridine or other quaternisable conjugated monomer units. In the case of devices using enzyme systems producing protons the polymer should have protonatable monomer units containing quaternisable nitrogen atoms - as for example in the specific examples mentioned in the preceding sentence.

The polymers may be prepared by any suitable method known in the art for the production of these materials in a suitable form with the desired conductivity. In general though the polymer may be made by electrochemical polymerisation in an electrochemical cell containing a solution of the monomer, together with a suitable amount of an electrical conductivity promoting dopant, in an electrochemically stable relatively polar solvent capable of maintaining ionic species in solution therein and thereby maintaining the conductivity of the solution in said electrochemical cell. Various suitable dopants are known in the art including strong protonic acids such as for example sulphonic acids, perchloric acid, and fluoroboric acid. The dopants may be employed in various proportions in the polymer especially in relation to conductivity, stability, and/or tractability as well as the nature of the individual dopant. Usually though the dopant is used in a molar ratio of from 4 to 2 : 1 relative to the polymer repeating unit, preferably from 4 to 3 : 1.

In general there is used an electrochemical cell having two electrodes, usually of highly conductive metal such as platinum, gold, or silver, across which is applied a voltage in the range from 0.5V above to 0.5V below the oxidation - reduction potential of the polymer. In practice a reference electrode e.g. a standard calomel electrode (SCE) is included in the cell to facilitate monitoring and control of the voltage at the electrode at which the polymer is formed to within the desired range. Where an SCE reference electrode is used the electrode at which the polymer is formed is generally maintained at a voltage of 0.5V to 1.5V relative to the SCE.

It will be appreciated that the elctrochemical polymerisation conditions such as applied voltage, temperature, solvent, monomer and dopant concentrations, rate of polymerisation, and electrode configuration may be varied to a greater or lesser extent to facilitate production of the polymer in a suitable form which will generally be a thin film. Suitable polymer, especially polypyrrole, thin films are commercially available, e.g. from the Polaroid Corporation of Cambridge, Mass., U.S.A. In the case though of soluble polymers which are soluble in organic solvents such as acetonitrile and dimethylformamide, the polymer can be initially produced in a different form e.g. a granular form where this is more convenient and then dissolved in a suitable solvent to form a solution which can be applied to a suitable substrate e.g. a printed circuit board or the like having conductor tracks thereon formed and arranged to provide the electrical connections from the polymer to a suitable resistance measuring means which may if desired also be mounted on said printed circuit board or the like.

The electrochemical polymerisation conditions may also be varied to adjust the conductivity of the polymer. In general the polymer should desirably have a conductivity in the range from $10^{-4}$ to $10^2$S cm$^{-1}$ depending upon such factors as the sensitivity of the resistance measuring means to be used with the device and the analyte concentration range to be measured. Further details of suitable processes for the preparation of conductive polymers are disclosed in for example F.M.Al-Arrayed et al in Materials Forum, 1986, 9, 209-216.

Various direct or indirect resistance/conductivity measuring means may be used to monitor the changes in conductivity of the polymer when the device is contacted with an analyte containing solution. Thus for example there could be used a standard ohm meter or resistance bridge device for measuring the resistance of the device and formed and arranged for indicating the absolute value of the resistance. In general though it will be more convenient to use a measuring means formed and arranged to display or otherwise output data indicating directly analyte concentrations corresponding to the changes in conductivity occurring.

It will be appreciated that the conductivity of some polymer film preparations is time dependent. Also the rate of response of the device of the invention in terms of establishing equilibrium conditions throughout the polymer element will vary significantly with inter alia the configuration of the polymer element and its surface area in contact with the enzyme support element, relative to the volume of said polymer element. Accordingly in use of the device it may be necessary on the one hand to measure the initial conductivity before the device is introduced to the analyte containing solution and at a suitable time after contact with the analyte containing solution to determine the relative change in conductivity, and on the other hand to monitor variation of conductivity over a period of time following initial exposure. In such cases the device would advantageously be used with suitable data recording and/or data processing means adapted to correlate the differences and/or variations in conductivity to analyte concentration in accordance with previously established relationships based on determinations of known analyte solutions.

Various enzyme catalyzed reaction systems are known in the art which generate protons or hydrogen peroxide in enzyme catalyzed reactions of analytes either directly or with derivatives obtained by further enzyme catalysed reactions. One suitable system for the determination of alcohol analytes uses an alcohol dehydrogenase to catalyse oxidation of the alcohol e.g. ethanol to acetaldehyde, by NAD with the liberation of protons. A system suitable for the determination of lactic acid uses lactate dehydrogenase to catalyse oxidation of the lactic acid to pyruvic acid by NAD. Another system using steroid dehydrogenases and NAD can also be used to determine certain steroids. Another system which is suitable for glutamic acid determination uses glutamate dehydrogenase to catalyse oxidation of the glutamic acid to 2-oxoglutamic acid by NAD with the liberation of protons. An enzyme system suitable for the determination of glucose comprises glucose oxidase which catalyses oxidation of glucose to gluconic acid with the liberation of hydrogen peroxide. Another system of this type can be used to determine cholesterol using the enzyme cholesterol oxidase again with the liberation of hydrogen peroxide. Certain amines and amino acids can be similarly detected using the appropriate amine or amino acid oxidase. In addition other analytes may be detected through the use of enzymes linked to antibodies to said analytes so that binding of the analyte with the antibody converts the enzyme from a catalytically inactive form to a catalytically active form which can then catalyse release of protons or hydrogen peroxide by a suitable reactant. Thus for example analytes such as cytotoxic drugs e.g. methotrexate; food toxins e.g. aflatoxins which are produced by moulds growing on peanuts, maize and wheat; and industrial effluents and toxic products, e.g. dioxin, may be determined using devices of the invention. Conveniently there may be used for example a system in which a suitable enzyme which catalyzes release of protons or hydrogen peroxide by suitable reagents, is conjugated to a sample of the desired analyte, by means of suitable protein modifying reactions e.g. using a carbodiimide to produce linking of carboxyl and amino groups, using reductive amination of aldehydes using sodiumcyanoborohydride and an amine; using acylation of amino groups with anhydrides; or any other suitable procedure known in the art. In the device ready for use the analyte-enzyme conjugate is bound by an antibody to the analyte. Thus when the device is contacted with a test solution containing analyte molecules, these bind the antibody displacing the previously bound enzyme-analyte conjugate freeing it to catalyze reaction of the proton or hydrogen peroxide releasing reagent. In one example that may be mentioned there could be used glucose oxidase enzyme with glucose reagent which releases hydrogen peroxide upon the glucose oxidase enzyme catalysed oxidation of glucose to gluconic acid.

As indicated above the enzyme support element is advantageously coated with a filter means for screening said enzyme support element and the polymer element to a greater or lesser extent from other materials present in the liquid sample to be tested which could interact with one or more of the enzyme, the enzyme catalysed reaction, and the polymer element. This is particularly desirable where the liquid sample is a biological fluid such as urine or a blood fraction which contains a whole variety of different substances. The filter means used will be substantially impermeable to proteinaceous material and/or macromolecular substances. Suitable filter means include highly crosslinked polymeric water-swellable gels such as polyacrylamide and polysaccharose gels.

The filter means is conveniently in the form of a layer secured to the outer face of the conducting polymer film for example by polymerisation thereon in the case of a polymeric gel or by mechanical securing with the aid of external mechanical supports or restraints. Thus for example, the device may conveniently be mounted in a housing of an inert and impermeable electrically insulating material e.g. a plastics material such as polyvinylchloride, with a 'window' opening across a central area of the outer face of the filter means. In use of the device of the invention an unknown solution believed to contain the analyte is contacted with the second face of the enzyme support element through the filter means and the change in conductivity between the electrical connection means measured by connection thereto of a resistance measuring means.

Thus in a further aspect the present invention provides a method of detecting a predetermined analyte in a liquid sample comprising the steps of contacting the liquid sample with the second face of the enzyme support element of the device of the invention through the filter means, selectively permeable to the analyte, and measuring the change in resistance between the connection means of the polymer element of said device.

Further preferred features and advantages of the invention will appear from the following detailed description given by way of example of a preferred embodiment illustrated with reference to the accompanying drawing in which :

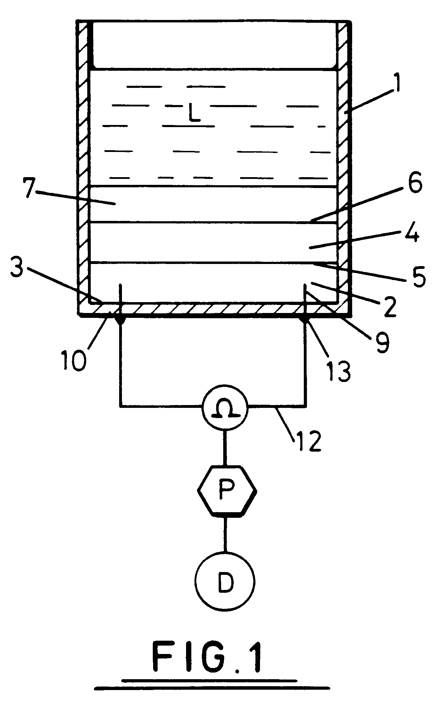

Fig. 1 is a generally schematic sectional elevation of a device of the invention in use in measuring the ethanol content of a liquid sample L.

Fig. 1 shows a device of the invention comprising a vessel 1 of an inert electrically insulating material such as glass having an electrically conducting polypyrrole layer 2 in the bottom 3 thereof. An ethanol permeable substantially electrically non-conducting enzyme support layer 4 is disposed on top of said polypyrrole layer 2 with a first face 5 in contact with the latter and a second face 6 covered in turn by a filter means layer 7. Two spaced apart electrical connections in the form of platinum wires 9 extend through the bottom wall 10 of the vessel 1 into the polypyrrole layer 2 and are connected 12 at their distal ends 13 to an ohm meter Ω

The enzyme support layer comprises a highly crosslinked polymeric water-swellable gel such as a polyacrylamide or polysaccharose gel to which is secured the enzyme(s) to be used together with any necessary cofactors. In the case of the device illustrated there is first formed a prepolymer of polyacrylamide having a molecular weight in the range from 1000 to 2000 to which is added ethanol dehydrogenase, and the co-enzymes NAD. The prepolymer is then cross-linked in conventional manner to entrap the enzyme and co-enzyme.

In order to convert the resistance measurements obtained from the ohm meter Ω, a data processing unit D is connected thereto. This unit is adapted to record differences in resistance measurements between successive readings and process these in accordance with previously established relationships obtained using known solutions to provide an output signal corresponding to the ethanol concentration present which is then displayed on a suitable visual display unit D.

EXAMPLE 1 - Construction of Ethanol Sensor

Polypyrrole Film (2.5 x 1.1 cms. 0.2mm thick) was mounted on a glass slide support using two metal conductor legs secured to the film in spaced apart relation, with the aid of a suitable non-conducting resin adhesive such as that available under the Trade Name "Araldite" from the Ciba-Geigy Company of Basel Switzerland. This resin was also used to provide an insulating coating for the legs between the slide and the film. Suitable conductor leads for attachment to a conductivity or ohm meter were connected to the metal conductor legs on the slide. The film element was then placed in a vessel to which was added activated polyacrylamide prepolymer (PAN) (1.5g containing approximately 1000 μ mol active esters) prepared in accordance with the procedures of Whitesides (A.Pollak et al J. Chem. Soc. 1980 Vol. 102 p.6324) dissolved in a buffer solution comprising 0.3 M Hepes buffer pH 7.5, 30 mM magnesium chloride and 0.3 mM NAD (8 ml) during 2-3 min. Dithiothreitol (0.1 ml) was added followed by triethylenetetraamine (0.85 ml) to initiate cross-linking. After 2.5 minutes a solution of horse liver alcohol dehydrogenase (40 mg) in the above buffer (1 ml) was added. The polymer began to gel 2 minutes later and the polypyrrole film coated with the enzyme system - containing polyacrylamide gel was withdrawn. The coated film was then allowed to dry in air at room temperature for 1 hour and was then washed by immersion successively in solutions of the above buffer containing ammonium sulphate (50 mM) for 20 minutes and finally in buffer solution alone. The washed coated polymer film was allowed to dry overnight after which the sensor was ready- for use.

EXAMPLE II - Preparation of Glucose Sensor

The above described procedure for preparation of an ethanol sensor was repeated with a separate sample of polypyrrole film except that glucose oxidase (10 mg) was added in place of the horse liver alcohol dehydrogenase, and nicotinamide adenine and dinucloetide (NAD)was omitted from the buffer solution.

EXAMPLE III - Detection of ethanol

The Ethanol Sensor of Example I was first deprotonated by immersion in aqueous sodium hydroxide (1M) for 5 minutes. (If desired lower concentrations e.g. 0.5M can be used provided the immersion time is extended appropriately - to 30 minutes in this case.) The Ethanol Sensor was then equilibrated by immersion in deionised water, removed from the water, superficial water shaken off, and the conductivity of the sensor then determined using a Solartron Schlumberger 1186 Electrochemical interface (available from the Solartron Limited of Farnborough, Hampshire, England). Ethanol (100 μl) was added to deionised water to give an ethanol concentration of 10 mM and the sensor immersed in the solution. The procedure for measuring conductivity was repeated after successfive periods of 2 minutes immersion.

Results

After 10 minutes, the current had risen by 0.9 mA and reached a maximum of 2.0 mA after 30 minutes. A further sample of ethanol (100 μl) was added to give a solution concentration of 20 mM and the experiment repeated. The current increase was 0.9 mA after 10 minutes and 2.8 mA after 30 minutes. A further sample of ethanol (100 μl) was added to give a solution concentration of 30 mM and the experiment repeated. The current increase was 1.2 mA after 10 minutes and 2.8 mA after 30 minutes. The initial deprotonation step described is in fact optional but does have the advantage of significantly increasing the sensitivity and responsiveness of the sensor. It is also possible to use a polypyrrole film in the sensor which film is in a substantially deprotonated form ab initio as a result of production thereof by electrochemical polymerisation at an overvoltage - preferably from 4.5 to 3.0V relative to SCE.

## EXAMPLE IV - Detection of glucose

The glucose Sensor was equilibrated by immersion in deionised water, the superficial water shaken off, and the conductivity determined as described in Example III. Glucose (100 mg) was added to give a solution concentration of 30 mM and the sensor immersed in the solution. The procedure for determining conductivity was repeated after successive periods of 2 minutes immersion.

## Results

After 4 minutes, the current increase was 30 mA and reached a maximum of 40 mA in 20 minutes. Following the same procedures but using two slightly thinner films prepared independently under substantially identical conditions a test solution containing 25mM glucose yielded current increases after 20 minutes of 37ma and 40 ma.

## EXAMPLE V - Preparation of cholesterol sensor

The procedure in Example I for the preparation of an ethanol sensor was followed except that cholesterol oxidase (1 mg, Sigma Chemical Co. Ltd., Poole, England) replaced alcohol dehydrogenase and nicotinamide adenine dinucleotide. The gelling time was $2\frac{1}{2}$ min and crosslinking time was 2 minutes.

## EXAMPLE VI - Detection of cholesterol

The cholesterol sensor was equilibrated as in Example III for the detection of ethanol. Its response to solutions of cholesterol water-containing 10% Brij 30 (a non-ionic polyethyleneoxide detergent available from I.C.I. plc of England)was determined.

## Results

On immersion in a solution of cholesterol (100 mM) the conductivity rose 3 mA in 15 minutes reaching a maximum of 6 mA in 70 minutes.

## Claims

1. A device for use in the detection, in a liquid sample, of an analyte which is reactive under predetermined enzyme-catalysed conditions so as to produce at least one of a proton and hydrogen peroxide, which device is in the form of a multilayer thin film device and comprises:
    (1) an electrically conducting thin film polymer element (2) which comprises a polymer including in its polymeric structure monomer-derived units having conjugatable electron systems and protonatable N atoms such that the polymer can exist in a protonated or deprotonated form; and being permeable to protons, and being permeable to hydrogen peroxide where said analyte under said enzyme-catalysed conditions produces hydrogen peroxide; and said electrically conducting polymer element having an electrical conductivity in the range of from $10^{-4}$ to $10^2$ S cm$^{-1}$ and which is variable according to the amount of protons or the amount of hydrogen peroxide in contact with said polymer element, in use the electrically conducting polymer element being initially in the reduced deprotonated form prior to contact with said liquid sample;
    (2) electrical connection means (9) being electrically-conductively attached to said electrically conducting polymer element, the electrical connection means being spaced apart one from another and being intended in use of the device for electrically connecting said electrically conducting polymer element to external electrical circuit means (12) to allow for directly or indirectly detecting changes in electrical resistance of said electrically conducting polymer element;
    (3) an enzyme support element (4) containing at least one enzyme, and additionally containing any cofactor(s) for said at least one enzyme which are required in said predetermined enzyme catalysed conditions, in an analyte-permeable substantially electrically non-conducting highly cross-linked polymeric water-swellable gel; the enzyme support element having a first face (5) which is in contact with said electrically conducting polymer element, and having a second face (6) which in use of the device may be brought indirectly into contact with said liquid sample containing said analyte to be detected; and
    (4) a filter means (7) selectively permeable to the analyte which is disposed at said second face (6) for screening said enzyme support element from at least one other component of said liquid sample and which is substantially impermeable to at least one of proteinaceous material and macromolecular substances, said filter means comprising a highly cross-linked polymeric water-swellable gel.

2. A device as claimed in claim 1 wherein is used an enzyme catalyst system (4) which produces protons when in contact with a said analyte.

3. A device as claimed in any preceding claim wherein the enzyme support element (4) contains an analyte specific dehydrogenase which in use of the device catalyses oxidation of the analyte with liberation of protons.

4. A device as claimed in claim 1 wherein the enzyme support element (4) contains an analyte specific oxidase which in use of the device catalyses oxidation of the analyte with liberation of hydrogen peroxide.

5. A device as claimed in any one of claims 1 to 4 wherein said polymer element (2), enzyme support element (4), and filter means (7) are mounted in a vessel (1) formed and arranged for holding, in use, a body of test solution (L) in contact indirectly through the filter means (7), with the second face (6) of the enzyme support element (4).

6. A device as claimed in any preceding claim wherein said enzyme support element (4) contains at least one enzyme linked to an antibody to said analyte, so that said enzyme is converted from an inactive to an active form upon reaction of said analyte with said antibody in use of the device, and wherein said enzyme support element (4) further includes at least one reagent reactive in the presence of said enzyme in its active form so as to produce at least one of a proton and hydrogen peroxide.

7. A device as claimed in any preceding claim having a said electrical circuit means connected to said connection means.

8. A method of detecting a predetermined analyte in a liquid sample comprising the steps of contacting the liquid sample (L) with the second face (6) of the enzyme support element (4) of a device (1) according to claim 1 through the filter means (7), selectively permeable to the analyte, and measuring the change in resistance between the connection means (9) of the polymer element (2) of said device (1).

**Revendications**

1. Dispositif utile pour détecter, dans un échantillon liquide, un analyte qui peut réagir dans des conditions catalysées par une enzyme prédéterminées en produisant au moins un proton ou du peroxyde d'hydrogène, lequel dispositif est sous la forme d'un dispositif à film mince multicouche et comprend:

(1) un élément (2) en polymère en film mince conduisant l'électricité, qui comprend un polymère incluant dans sa structure polymère des unités dérivées de monomère ayant des systèmes d'électrons pouvant être conjugués et des atomes de N pouvant être protonés de telle sorte que le polymère peut exister sous une forme protonée ou déprotonée; et étant perméable aux protons, et étant perméable au peroxyde d'hydrogène lorsque dans ces conditions catalysées par une enzyme, cet analyte produit du peroxyde d'hydrogène; et cet élément en polymère conduisant l'électricité ayant une conductivité électrique de l'ordre de $10^{-4}$ à $10^2$ S.cm$^{-1}$ et qui est variable en fonction de la quantité de protons ou de la quantité de peroxyde d'hydrogène en contact avec cet élément en polymère, en cours d'utilisation l'élément en polymère conduisant l'électricité étant initialement sous la forme déprotonée réduite avant mise en contact avec cet échantillon liquide;

(2) des moyens (9) de connection électrique étant rattachés à cerf élément en polymère conduisant l'électricité de façon à conduire l'électricité, les moyens de connexion électrique étant espacés l'un de l'autre et conçus pour raccorder électriquement, lors de l'emploi du dispositif, cet élément en polymère conduisant l'électricité à un circuit électrique externe (12) pour permettre la détection directe ou indirecte des changements de résistance électrique de cet élément en polymère conduisant l'électricité;

(3) un élément (4) de support d'enzyme contenant au moins une enzyme et contenant de plus un ou de quelconques cofacteurs pour cette enzyme au moins présente, qui sont nécessaires dans ces conditions prédéterminées catalysées par une enzyme, dans un gel polymère fortement réticulé pouvant gonfler dans l'eau, perméable aux analytes et ne conduisant pratiquement pas l'électricité; l'élément de support d'enzyme ayant une première face (5) qui est en contact avec cet élément en polymère conduisant l'électricité et ayant une seconde face (6) qui, lors de l'utilisation du dispositif, peut être mise indirectement en contact avec cet échantillon liquide contenant cet analyte à détecter; et

(4) un moyen de filtration (7) perméable sélectivement à l'analyte, qui est disposé sur cette seconde face (6) pour protéger cet élément de support d'enzyme contre au moins un autre composant de cet échantillon liquide et qui est notablement imperméable à au moins une matière parmi une matière protéinique et des substances macromoléculaires, ce moyen de filtration comprenant un gel polymère fortement réticulé pouvant gonfler dans l'eau.

2. Dispositif suivant la revendication 1, dans lequel on utilise un système catalytique d'enzyme (4) qui produit des protons lorsqu'il est en contact avec cet analyte.

3. Dispositif suivant la revendication 1 ou la revendication 2, dans lequel l'élément de support d'enzyme (4) contient une déshydrogénase spécifique de l'analyte qui, lors de l'utilisation du dispositif, catalyse l'oxydation de l'analyte avec une libération de protons.

4. Dispositif suivant la revendication 1, dans lequel l'élément de support d'enzyme (4) contient une oxydase spécifique de l'analyte qui, lors de l'utilisation du dispositif, catalyse l'oxydation de l'analyte avec une libération de peroxyde d'hydrogène.

5. Dispositif suivant l'une quelconque des revendications 1 à 4, dans lequel cet élément (2) de polymère, cet élément de support d'enzyme (4) et ce moyen de filtration (7) sont montés dans un récipient (1) formé et disposé pour maintenir, lors de l'utilisation, un corps de solution d'essai (L) indirectement en contact à travers le moyen de filtration (7), avec la seconde face (6) de l'élément de support d'enzyme (4).

6. Dispositif suivant l'une quelconque des revendications précédentes, dans lequel cet élément de support d'enzyme (4) contient au moins une enzyme liée à un anticorps pour cet analyte, de telle sorte que cette enzyme est convertie depuis une forme inactive en une forme active lors de la réaction de cet analyte avec cet anticorps lors de l'utilisation du dispositif, et dans lequel cet élément de support d'enzyme (4) comprend de plus un réactif pouvant réagir en présence de cette enzyme sous sa forme active de façon à produire au moins un proton ou du peroxyde d'hydrogène.

7. Dispositif suivant l'une quelconque des revendications précédentes comprenant un circuit électrique relié à ces moyens de connexion.

8. Méthode pour détecter un analyte prédéterminé dans un échantillon liquide comprenant les étapes de mise en contact de l'échantillon liquide (L) avec la seconde face (6) de l'élément de support d'enzyme (4) d'un dispositif (1) selon la revendication 1 à travers le moyen de filtration (7) perméable sélectivement à l'analyte et la mesure du changement de résistance entre les moyens de connexion (9) de l'élément de polymère (2) de ce dispositif (1).

**Patentansprüche**

1. Vorrichtung zur Verwendung beim Nachweis von einer in einer flüssigen Probe befindlichen analytisch zu bestimmenden Verbindung, welche unter vorbestimmten enzymkatalysierten Bedingungen reaktiv ist, indem sie mindestens entweder ein Proton oder Wasserstoffperoxid liefert, in Form eines mehrschichtigen dünnen Films, umfassend:

(1) ein elektrisch leitendes Element aus polymerem Dünnfilm (2), welches ein Polymer mit in seiner Polymer-Struktur eingeschlossenen Einheiten aus Monomer-Derivaten mit konjugierbaren Eletronen-Systemen und protonierbaren N-Atomen umfaßt so, daß das Polymer in protonierter und entprotonierter Form vorliegen kann, wobei das elektrisch leitende Polymerelement für Protonen und Wasserstoffperoxid durchlässig ist, wenn die zu analysierende Verbindung unter den enzymkatalysierten Bedingungen Wasserstoffperoxid produziert, und wobei das elektrisch leitende Polymerelement eine elektrische Leitfähigkeit im Bereich von $10^{-4}$-$10^2$ S cm$^{-1}$ aufweist, welche gemäß dem mit dem Polymerelement in Kontakt befindlichen Gehalt an Protonen oder Wasserstoffperoxid variabel ist und wobei das elektrisch leitende Polymerelement bei Anwendung anfangs in reduzierter entprotonierter Form vorliegt, bevor es mit der flüssigen Probe in Kontakt kommt;

(2) voneinander beabstandete elektrische Verbindungen (9), die elektrisch leitend an das elektrisch leitende Polymerelement angeschlossen sind und welche das elektrisch leitende Polymerelement beim Gebrauch der Vorrichtung an externe elektrische Leitungen (12) anschließen sollen, um elektrische Widerstandsänderungen des elektrisch leitenden Polymerelements direkt oder indirekt zu bestimmen;

(3) ein Trägerelement für Enzyme (4) mit mindestens einem Enzym und zusätzlich sämtlichen für die vorbestimmten enzymkatalysierten Bedingungen erforderlichen Kofaktoren für mindestens dieses eine Enzym in einem für die zu analysierende Verbindung durchlässigen, im wesentlichen elektrisch nicht leitenden, in hohem Grade quervernetzten, polymeren, in Wasser quellbaren Gel, wobei das Enzymträgerelement eine im Kontakt mit dem elektrisch leitenden Polymerelement stehende erste Oberfläche (5) besitzt und eine zweite Oberfläche (6), welche bei Anwendung der Vorrichtung indirekt mit der flüssigen, die zu bestimmende Substanz enthaltenden Probe in Kontakt steht; und

(4) eine für die zu analysierende Verbindung selektiv durchlässige Filtervorrichtung (7), welche an der zweiten Oberfläche (6) anliegt, um das Enzymträgerelement gegen mindestens eine andere Komponente der flüssigen Probe abzuschirmen und welche im wesentlichen undurchlässig für mindestens entweder proteinhaltige Bestandteile oder für hochmolekulare Substanzen ist, wobei die Filtervorrichtung mindestens ein in hohem Grade quervernetztes, polymeres, in Wasser quellbares Gel umfaßt.

2. Vorrichtung nach Anspruch 1, in welchem ein Enzym-Katalysator-System (4) verwendet wird, welches Protonen liefert, sobald es in Kontakt mit der zu analysierenden Verbindung kommt.

3. Vorrichtung nach jedem der vorstehenden Ansprüche, in welchem das Enzymträgerelement (4) eine für die zu analysierende Verbindung spezifische Dehydrogenase enthält, welche bei Anwendung der Vorrichtung die Oxidation der zu analysierenden Verbindung unter Freisetzung von Protonen katalysiert.

4. Vorrichtung gemäß Anspruch 1, in welchem das Enzymträgerelement (4) eine für die zu analysierende Verbindung spezifische Oxidase enthält, welche bei Anwendung der Vorrichtung die Oxidation der zu analysierenden Verbindung unter Freisetzung von Wasserstoffperoxid katalysiert.

5. Vorrichtung nach jedem der Ansprüche 1-4, in welcher das Polymerelement (2), das Enzymträgerelement (4) und die Filtervorrichtung (7) in einem Gefäß (1) untergebracht sind, welches in Form und Anordnung so ausgebildet ist, daß es die Testlösung (L) über die Filtervorrichtung (7) mit der zweiten Oberfläche (6) des Enzymträgerelements (4) indirekt in Kontakt hält.

6. Vorrichtung nach jedem der vorhergehenden Ansprüche, in welcher das Enzymträgerelement (4) mindestens ein an einen Antikörper der zu analysierenden Verbindung gekoppeltes Enzym enthält, so, daß bei Anwendung der Vorrichtung nach Reaktion der zu analysierenden Verbindung mit dem Antikörper das Enzym aus einer inaktiven in eine aktive Form überführt wird, und in welcher das Enzymträgerelement (4) ferner mindestens ein in Gegenwart des Enzyms in seiner aktiven Form reaktives Reagens umfaßt, so daß mindestens entweder ein Proton oder Wasserstoffperoxid produziert wird.

7. Vorrichtung nach jedem der vorhergehenden Ansprüche mit an die Anschlüsse angeschlossenen elektrischen Leitungen.

8. Verfahren zum Nachweis einer zu analysierenden vorbestimmten Verbindung in einer flüssigen Probe in folgenden Schritten: In Kontakt bringen einer flüssigen Probe (L) mit der zweiten Oberfläche (6) des Enzymträgerelements (4) einer Vorrichtung (1) gemäß Anspruch 1 über die für die zu bestimmende Verbindung selektiv durchlässige Filtervorrichtung (7) und Messen der Widerstandsänderung zwischen den Anschlußverbindungen (9) des Polymerelements (2) der Vorrichtung (1).

# FIG.1